Europäisches Patentamt

European Patent Office

Office européen des brevets

(11) Numéro de publication: **0 138 667 B1**

# (12) FASCICULE DE BREVET EUROPEEN

(45) Date de publication de fascicule du brevet:
21.11.91

(51) Int. Cl.⁵: **G01N 33/569**

(21) Numéro de dépôt: **84401834.1**

(22) Date de dépôt: **14.09.84**

(54) **Méthode pour le diagnostic de lymphadénopathie et du syndrome d'immuno-dépression acquise.**

(30) Priorité: **15.09.83 GB 8324800**

(43) Date de publication de la demande:
**24.04.85 Bulletin 85/17**

(45) Mention de la délivrance du brevet:
**21.11.91 Bulletin 91/47**

(84) Etats contractants désignés:
**AT BE CH DE FR GB IT LI LU NL SE**

(56) Documents cités:

SCIENCE, vol. 220, no. 4599, 20 mai 1983, pages 868-871, Washington D.C., US; F. BARRE-SINOUSSI et al.: "Isolation of a T-Lymphotropic retrovirus from a patient at risk for acquired immune deficiency syndrome (AIDS)"

NATURE, vol. 309, no. 5929, 3 mai 1984, pages 12,13, Londres, GB; R. WEISS: "Retroviruses linked with AIDS"

(73) Titulaire: **INSTITUT PASTEUR Fondation reconnue d'utilité publique**
**28 rue du Docteur Roux**
**F-75724 Paris Cédex 15(FR)**

(72) Inventeur: **Montagnier, Luc**
**21, rue de Malabry**
**F-92350 Le Plessis-Robinson(FR)**
Inventeur: **Cherman, Jean-Claude**
**2, Clos d'Orgal**
**F-78310 Elancourt(FR)**
Inventeur: **Barre-Sinoussi, Françoise**
**104, le Capricorne 50, rue d'Erevan**
**F-92130 Issy-Les-Moulineaux(FR)**
Inventeur: **Vizinet-Brun, Françoise**
**24, Boulevard Saint-Germain**
**F-75005 Paris(FR)**
Inventeur: **Rouzioux, Christine**
**21, rue de Dantzig**
**F-75015 Paris(FR)**
Inventeur: **Rosenbaum, Willy**
**11, Passage de la Main d'Or**
**F-75011 Paris(FR)**
Inventeur: **Dauguet, Charles**
**19, rue Jean-Jacques Rousseau**
**F-75001 Paris(FR)**

SCIENCE, vol. 215, no. 4535, 19 février 1982, pages 975-978, AAAS, Washington D.C., US; M. ROBERT-GUROFF et al.: "Natural antibodies to human retrovirus HTLV in a cluster of Japanese patients with adult T cell leukemia"

NATURE, vol. 294, no. 5836, 19 novembre 1981, pages 271-273, MacMillan Journals LTD., Londres, GB; V.S. KALYANARAMAN et a.: "Antibodies in human sera reactive against an internal structural protein of human T-cell lymphoma virus"

SCIENCE, vol. 225, 1984, pp. 63-66

Inventeur: **Gruest, Jacqueline**
6, rue du Gué
F-94240 l'Hay Les Roses(FR)
Inventeur: **Nugeyre, Marie-Thérèse**
6, rue Dombasle
F-75015 Paris(FR)
Inventeur: **Ray, Françoise**
10, rue d'Odessa
F-75014 Paris(FR)
Inventeur: **Axler-Blin, Claudine**
137, rue Lecourbe
F-75015 Paris(FR)
Inventeur: **Chamaret, Solange**
324, rue Lecourbe
F-75015 Paris(FR)

(74) Mandataire: **Gutmann, Ernest et al**
S.C. Ernest Gutmann - Yves Plasseraud 67,
boulevard Haussmann
F-75008 Paris(FR)

## Description

La présente invention est relative à un procédé mettant en oeuvre un antigène purifié et à des moyens et des méthodes pour le diagnostic de lymphadénopathie et du syndrome d' immuno-dépression acquise mettant en oeuvre cet antigène.

Le syndrome d'immuno-dépression acquise (SIDA) a été récemment reconnu dans plusieurs pays. Il a été décrit que la maladie se trouve principalement chez des hôtes mâles homosexuels ayant plusieurs partenaires et des études épidémiologiques suggèrent une transmission horizontale par des voies sexuelles ainsi que par l'administration de médicaments par voie intraveineuse et la transfusion sanguine. La dépression notable de l'immunité cellulaire qui apparaît chez les malades atteints de SIDA et les modifications quantitatives des sous-populations de leurs lymphocytes T suggèrent que les cellules T ou qu'un sous-ensemble T pourraient constituer une cible préférentielle pour l'agent infectieux supposé. Dans une variante, ces modifications peuvent résulter d'infections ultérieures. L'immuno-dépression cellulaire peut se traduire par de sérieuses infections éventuelles chez des malades souffrant de SIDA, nombreux étant ceux qui développent le sarcome de Kaposi. Cependant, il a également été constaté un syndrome de lymphadénopathies multiples persistentes chez des homosexuels masculins et des jeunes enfants qui peuvent ou ne peuvent pas développer le SIDA. L'aspect histologique de tels ganglions lymphatiques est celui de l'hyperplasie réactive. De tels cas peuvent correspondre à une forme précoce ou à une forme plus douce de la maladie.

Il a été constaté que l'un des agents étiologiques majeurs du SIDA et du syndrome de lymphadénopathie (SLA) qui est souvent considéré comme étant un signe prodomique du SIDA, devrait consister en un rétrovirus T-lymphotrope qui a été isolé à partir d'un ganglion lymphatique d'un malade homosexuel souffrant de lymphadénopathies multiples.

Le premier isolement chez un malade d'un tel rétrovirus a été décrit par F. Barré-Sinoussi et al, 20 mai 1983, SCIENCE, vol. 220, 868-870. Ce rétrovirus semble être distinct de la famille des rétrovirus humains, de la leucémie T (HTLV) (R.C. Gallo et M. S. Reitz. "J. Natl. Cancer Inst." 69 (No 6), 1209 (1982)). Il est connu que les derniers rétrovirus mentionnés appartiennent au sous-groupe dénommé HTLV-1.

Le malade susdit était un homosexuel de 33 ans, qui venait consulter un médecin en décembre 1982 pour une lymphadénopathie cervicale et une asthénie (malade 1). L'examen avait révélé des lymphadénopathies axillaires et inguinales. On n'avait noté ni fièvre ni perte récente de poids. Le malade avait un antécédent de plusieurs épisodes de gonorrées et avait été traité pour la syphilis en septembre 1982. Pendant les entretiens, il a indiqué qu'il avait eu plus de 5O partenaires sexuels par an et qu'il avait voyagé dans de nombreux pays, y compris l'Afrique de Nord, la Grèce et l'Inde. Son dernier voyage à New-York remontait à 1979.

Des tests de laboratoire indiquaient une sérologie positive (immunoglobuline G) pour le cytomégalovirus (CMV) et le virus d'Epstein-Barr. On avait détecté le virus d'herpès simplex dans des cellules provenant de sa gorge, et on les avait cultivées sur des cellules humaines et de singe. Une biopsie d'un ganglion lymphatique cervical avait été réalisé. Un échantillon a servi pour un examen histologique, qui a révélé une hyperplasie folliculaire sans changement de la structure générale du ganglion lymphatique. Des études immunohistologiques ont révélé, dans des régions paracorticales, de nombreux lymphocytes T (OKT3$^+$). La détermination de la nature d'une suspension cellulaire entière a indiqué que 62 % des cellules étaient des lymphocytes T (OKT3$^+$), 44 % étaient des cellules T-auxiliaires (OKT4$^+$) et 16 % étaient des cellules de répression (OKT8$^+$).

Des cellules provenant du même ganglion lymphatique biopsié ont été introduites dans un milieu de culture avec de la phytohémagglutine (PHA), du facteur de croissance de cellule T (TCGF) et de l'antisérum d' α interféron humain ("On a fait croître les cellules dans un milieu RPMI-164O complété avec des antibiotiques, $10^{-5}$ Mde β-mercaptoéthanol, 1O % de sérum de veau fétal, 0.1 % d'anticorps de mouton anti-α-interféron humain (titre neutralisant, 7 IU à une dilution de $10^{-5}$ et 1O % de TCGF, dépourvu de PHA"). On a utilisé l'antisérum anti-α-interféron afin de neutraliser l'interféron endogène qui est sécrété par des cellules infectées de façon chronique par des virus y compris des rétrovirus. Dans le système de la souris, il avait été préalablement montré que l'antisérum de l'interféron pouvait accroître la production de rétrovirus selon un facteur de 1O à 5O (F. Barré-Sinoussi et coll., "Ann. Microbiol. (Institut Pasteur)" 13OB, 349 (1979). Après 3 jours, on a poursuivi la culture dans le même milieu sans PHA. Des échantillons ont été prélevés régulièrement pour le titrage de la reverse transcriptase et l'examen au microscope électronique.

Après 15 jours de culture on a détecté une activité de reverse transcriptase dans le liquide surnageant de la culture, en utilisant les conditions ioniques décrites pour HTLV- 1[B.J. Poiesz et coll. "Proc. Natl. Acad. Sci. U.S.A." 77, 7415 (198O)]. La production de virus a continué pendant 15 jours puis a diminué, parallèlement au déclin de la prolifération de lymphocyte. Des lymphocytes sanguins périphériques cultivés

3

de la même façon ont été négatifs de façon constante, en ce qui concerne l'activité de la reverse transcriptase, même après 6 semaines. Le cytomégalovirus a pû être détecté, après co-culture prolongée avec des cellules MRC5, dans le tissu de biopsie d'origine mais non dans les lymphocytes T cultivés à un quelconque moment de la culture.

Le virus nouvellement isolé, qui sera dénommé par la suite $LAV_1$, sera cependant décrit d'abord.

Le virus est transmissible à des cultures de lymphocytes T obtenues à partir de donneurs en bonne santé. On a essayé en particulier de réaliser une transmission de virus en utilisant une culture de lymphocytes T établie à partir d'un donneur adulte en bonne santé du Centre de Transfusion Sanguine de l'Institut Pasteur.Au troisième jour, la moitié de la culture a été cultivée avec des lymphocytes provenant de la biopsie après centrifugation des suspension de cellules mélangées. On a pu détecter l'activité de la reverse transcriptase dans le surnageant après 15 jours de co-culture, mais non après 5 et 10 jours. La reverse transcriptase avait les mêmes caractéristiques que celle qui était libérée par les cellules du malade et la quantité libérée est restée stable pendant 15 à 20 jours. Des cellules de la culture non infectée des lymphocytes du donneur n'ont pas libéré d'activité de reverse transcriptase pendant cette période ou jusqu'à une période atteignant 6 semaines, après arrêt de la culture.

Le surnageant dépourvu de cellules de la coculture infectée a été utilisé pour infecter des cultures de 3 jours de lymphocytes T provenant de deux cordons ombilicaux, LC1 et LC5, en présence de Polybrène (2 $\mu$g/ml). Après une période de latence de 7 jours, on a détecté un titre relativement élevé d'activité de reverse transcriptase dans le surnageant des deux cultures de lymphocytes de cordons. Des cultures identiques qui n'avaient pas été infectées sont restées négatives. Ces deux infections successives montrent clairement que le virus pouvait être propagé sur des lymphocytes normaux provenant soit de nouveaux-nés soit d'adultes.

Dans les co-cultures ci-dessus, on a utilisé soit les cellules du malade 1 telles que (elles ont décliné et ne sont pas développées davantage), soit des cellules qui ont été soumises à un pré-traitement aux rayons X ou traitées par la mitomycine C.

Le virus $LAV_1$ ou des suspensions de virus $LAV_1$ qui peuvent être obtenues à partir de cultures infectées de lymphocytes, présentent des caractéristiques qui les distinguent complètement d'autres HTLV. Ces caractéristiques seront rapportées par la suite et lorsque cela est approprié, par rapport au dessin. Celui-ci montre des courbes représentatives de la variation de l'activité de la reverse transcriptase et de l'activité de la[$^3$H] uridine respectivement en fonction de fractions successives du virus $LAV_1$ dans un gradient de sucrose, après ultracentrifugation dans celui-ci des virus contenus dans un surnageant dépourvu de cellule, obtenu à partir d'une culture de lymphocytes infectées.

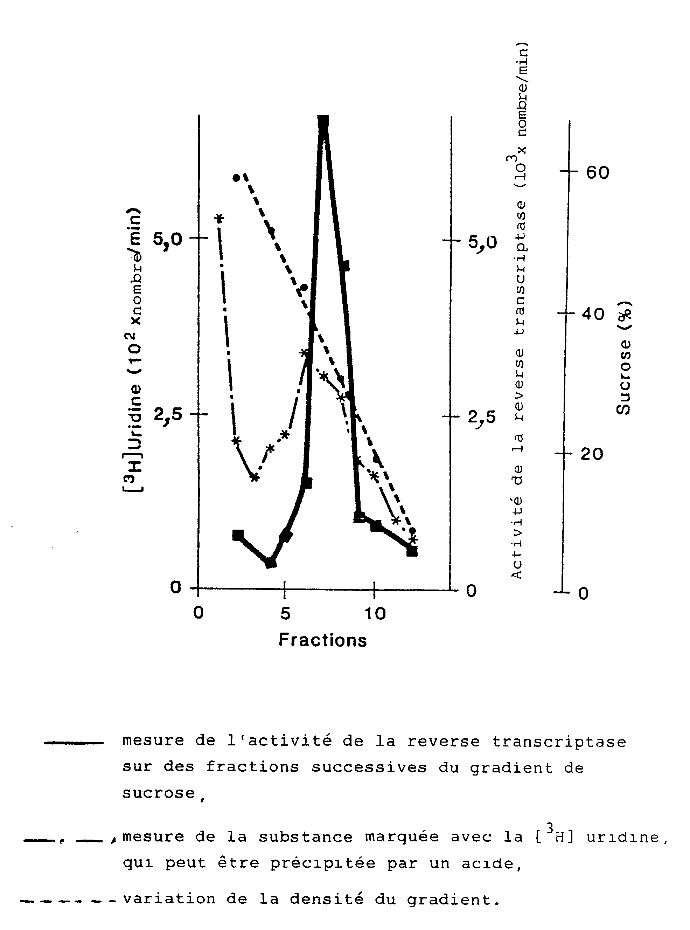

L'analyse du virus $LAV_1$ par l'intermédiaire de l'activité de la reverse transcriptase peut être réalisée selon la procédure qui était utilisée à propos du virus provenant du malade 1, sur la figure 1. Les résultats de l'analyse sont illustrés dans la figure 1. On a marqué des lymphocytes T du sang de cordon avec un virus provenant du malade 1, pendant 18 heures avec de la [$^3$H] uridine (28 Ci/mmole, Amersham; 20 $\mu$Ci/ml). On a soumis le surnageant dépourvu de cellules à une ultracentrifugation pendant 1 heure à 50,000 Tours/min. On a remis en suspension le culot dans 200 $\mu$l de tampon NTE (10 mM de tris, pH 7,4, 100 mM de NaCl, et 1 mM d'EDTA) et on a centrifugé sur 3 ml d'un gradient de sucrose linéaire (10 à 60 %) à 55,000 Tours/min. pendant 90 minutes dans un rotor IEC type SB 498. On a recueilli des fractions (200 $\mu$l), et on a titré des échantillons de 30 $\mu$l de chaque fraction pour déterminer l'activité de la polymérase fonction de l'ADN-ARN avec 5mM de $Mg^{2+}$ et du poly (A)-oligo-$(dT)_{12-18}$ comme matrice d'initiation ; on a fait précipiter une portion de 20 $\mu$l de chaque fraction avec de l'acide trichloroacétique à 10 % puis on a filtré sur un filtre Millipore de 0.45 $\mu$m. On a mesuré la substance $^3$H-marquée pouvant être précipitée par un acide dans un compteur $\beta$-Packard.

Le fait que le nouvel isolat de virus était un rétrovirus a été à nouveau indiqué par sa densité dans le gradient de sucrose ci-dessus qui était de 1.16, et par son marquage avec la [$^3$H] uridine (fig. 1). Un ARN sédimentant rapidement semble être associé au virus $LAV_1$.

Des cellules infectées par le virus provenant de la biopsie d'origine ainsi que des lymphocytes infectés provenant du premier et du second passage viral ont été utilisés pour déterminer les conditions optimales pour l'activité de la reverse transcriptase et la spécificité de matrice de l'enzyme. Les résultats ont été les mêmes dans tous les cas. L'activité de la reverse transcriptase s'est manifestée par une forte affinité pour le poly(adénylate-oligodéoxy-thymidylate) [poly(A)-oligo$(dT)_{12-18}$] et a nécessité du $Mg^{2+}$ avec une concentration optimale (5mM) et un pH optimal de 7,8. La réaction n'a pas été inhibée par l'actinomycine D. Ce caractère, ainsi que la spécificité préférentielle pour le riboseadénylate-déoxythymidylate par rapport au déoxyadénylate-déoxythymidylate, distingue l'enzyme virale des polymérases dépendantes de l'ADN.

Une microscopie électronique de sections ultra fines de cellules produisant le virus montre deux types de particules, vraisemblablement correspondantes aux formes matures et immatures du virus : des

4

particules immatures bourgeonnent à la surface de la cellule, avec un croissant dense en étroit contact avec la membrane du plasma. De temps en temps, certaines particules restent dans cet état, tout en étant détachées de la surface cellulaire.

Des particules matures présentent une morphologie tout-à-fait différente avec un noyau excentrique dense et petit (diamètre moyen : 41 nM). La plupart des virions sont ronds (diamètre moyen : 139 nM) ou ovoides, mais dans certaines photos, en particulier dans les particules vues dans la culture d'origine à partir de laquelle a été isolé le virus on peut également observer une morphologie caudale. Cette dernière forme peut également être notée dans des vacuoles cytoplasmiques qui sont libérés dans le milieu. De telles particules sont également formées par bourgeonnement à partir de membranes de vacuoles.

La morphologie des particules matures est nettement distincte de celle du HTLV, dont le grand noyau présente un diamètre moyen de 92 nM.

Les lymphocytes T-auxiliaires (cellules 3 Leu) forment la principale cible du virus. En d'autres termes, le virus $LAV_1$ a un tropisme particulier pour ces cellules. Des cellules Leu 3 sont reconnaissables par des anticorps monoclonaux industrialisés par ORTHO sous la dénomination commerciale OKT4. Au contraire, des cultures enrichies en cellules Leu 2, qui sont principalement des cellules cytotoxiques ou de répression et sont reconnues par les anticorps monoclonaux industrialisés par ORTHO sous la dénomination commerciale de OKT8 ne produisent, lorsqu'elles sont infectées dans les mêmes conditions, aucune activité détectable RT, même 6 semaines après l'infection par le virus.

Dans la plupart des cas de SIDA, le rapport des cellules de $OKT4^+$ sur $OKT8^+$, qui est normalement supérieur à 1, est réduit à des valeurs aussi faibles que 0,1 ou moins.

Le virus $LAV_1$ est également distinct du point de vue immunologique des isolats de HTLV-1 connus auparavant, provenant de lymphocytes T cultivés de malades souffrant de lymphomes T et de leucémies T. Les anticorps utilisés étaient spécifiques pour les protéines nucléaires p19 et p24 du HTLV-1. On a utilisé un anticorps monoclonal du p19 [M. Robert-Guroff et coll. "J. Exp. Med."154, 1957 (1981)] et un anticorps polyclonal de chèvre du p24 [V.S. Kalyanaraman et coll. "J. Virol.",38,906 (1981)] dans un titrage indirect de fluorescence vis-à-vis de cellules infectées provenant de la biopsie du malade 1 et de lymphocytes obtenus chez un donneur en bonne santé et infectés avec le même virus. Les cellules produisant le virus $LAV_1$ n'ont réagi avec aucun de ces types d'anticorps, tandis que deux lignées de lymphocytes de cordon infectées de façon chronique avec du HTVL 1[M. Popovic, P.S. Sarin, M. Robert-Guroff, V.S. Kalyanaraman, D. Mann, J. Minowada, R.C. Gallo "Science" 219, 856 (1983)] et utilisées comme témoins ont montré une forte fluorescence de surface.

Afin de déterminer quel antigène viral était reconnu par des anticorps présents dans les sérums du malade, on a effectué plusieurs expériences d'immunoprécipitations. Des lymphocytes de cordon infectés par le virus provenant du malade 1 et des témoins non infectés ont été marqués avec de la [$^{35}$S] méthionine pendant 20 heures. Les cellules ont été lysées avec des détergents et un extrait cytoplasmique S-10 a été fait. Le virus marqué libéré dans le surnageant a été séparé en bandes dans un gradient de sucrose. Les deux substances ont été immunoprécipitées par l'anti-sérum de HTVL-1 p24, par le sérum provenant du malade 1, et par des échantillons de sérum de donneurs en bonne santé. On a analysé des immunocomplexes par électrophorèse sur gel de polyacrylamide dans des conditions dénaturantes. On a reconnu de façon spécifique une protéine p25 présente dans les cellules infectées de virus, provenant du malade 1 et dans des cellules LC1 infectées avec ce virus, à l'aide du sérum provenant du malade 1 mais non à l'aide de l'anti-sérum de HTLV-1 p24 obtenu dans des conditions analogues ou du sérum de donneurs normaux. Réciproquement, le p24 présent dans les extraits de cellule infectée de HTLV de contrôle est reconnu par des anticorps du HTLV mais non par le sérum provenant du malade 1.

La principale protéine (p25) détectée après purification du virus marqué par la $^{35}$S-méthionine a un poids moléculaire d'environ 25,000 (ou 25K). Cette protéine est reconnue par le sérum du malade 1. Par analogie avec d'autres rétrovirus, on a considéré que cette protéine majeure est située dans le noyau viral.

Ceci peut être confirmé dans des expériences d'immuno-microscopie électronique, qui montrent que le sérum du malade peut agglutiner les noyaux viraux. Réciproquement, un antisérum suscité dans un lapin vis-à-vis d'un virus traité par de l'éther ne fait pas précipiter la protéine p25.

A côté de la protéine p25, d'autres protéines pouvaient être détectées par électrophorèse sur gel dans des conditions dénaturantes analogues, lorsque le virus avait au préalable été marqué de façon métabolique avec la $^{35}$S-cystéine. Ces protéines avaient des poids moléculaires d'environ 18,000 (p18) et 12,000 (p12). Les mêmes bandes de protéines sont également apparentes après coloration à l'argent. Ces protéines sont également reconnues par les sérums de malades souffrant de SLA ou de SIDA.

L'origine virale d'autres protéines vues dans l'électrophorèse sur gel de polyacrylamide du virus purifié est plus difficile à déterminer. On a pû voir une protéine p15 après coloration à l'argent, mais elle était beaucoup plus faible après marquage à la $^{35}$S-méthionine, vraisemblablement en raison de la rareté de cet amino-acide dans la protéine. Dans la gamme de poids moléculaire supérieure, une contamination du virus par des protéines cellulaires, soit à l'intérieur soit à l'extérieur de l'enveloppe virale est vraisemblable. Une protéine 36K, une protéine 42 K et une protéine 80 K sont formées constamment pour être associées au virus purifié et peuvent représenter les protéines majeures de l'enveloppe.

Une protéine p19 (ou ayant un poids moléculaire d'environ *9 mM) n'a pas été isolée à partir d'extraits de $LAV_1$.

L'invention concerne plus particulièrement un procédé de diagnostic in vitro d'un SLA ou d'un SIDA mettant en oeuvre une protéine p25 purifiée, telle qu'elle peut être obtenue à partir d'extraits ou de lysats de ce virus dès qu'ils peuvent être reconnus immunologiquement par des sérums de malades souffrant de SLA ou de SIDA. Il est inutile de dire que tout type de titrage immunologique peut être mis en jeu. A titre d'exemples, des titrages par immunofluorescence ou immunoenzymatiques ou des tests de radio-immunoprécipitation sont particulièrement appropriéS.

En pratique, et à l'exception de circonstances exceptionnelles, des sérums de patients affectés par la maladie ne reconnaissent pas le virus $LAV_1$ intact ou des virus ayant des propriétés phénotypiques ou immunologiques analogues. Dès que les protéines du noyau ont été exposées à ces sérums, la détection immunologique devient possible. La présente invention concerne en particulier un procédé de diagnostic in vitro mettant en oeuvre des extraits enrichis en protéines p25 ou même la protéine P25 purifiée ou une protéine immunogiquement apparentée à celle-ci. Toute procédure de purification peut être utilisée. A titre d'exemple seulement, on peut utiliser des procédures de purification telles que celles qui sont décrites par R.C. Montelaro et coll., J. of Virology, juin 1982, pp. 1029-1038.

On peut, de façon plus générale, mettre en oeuvre des protéines p25 purifiées obtenues à partir d'extraits ou lysats d'un quelconque virus ayant un phénotype analogue et apparenté immunologiquement à $LAV_1$. Des sources de virus du type LAV comprennent des cultures de lymphocytes T, pouvant être isolées à partir de malades souffrant de SLA et de SIDA ou à partir d'hémophiles.

A ce propos, d'autres extraits préférés sont ceux qui ont été obtenus à partir de rétrovirus obtenus par propagation sur des lymphocytes normaux des rétrovirus isolés à partir :

1) de lymphocytes de ganglions lymphatiques d'un homosexuel caucasien ayant eu plusieurs partenaires, et présentant d'importantes lésions du type sarcome de Kaposi et une sérieuse lymphopénie avec une déplétion pratiquement totale de son sang en lymphocyte $OKT4^+$ ;

2) de lymphocytes sanguins d'un jeune hémophile B présentant une neurotoxoplasmose et un rapport $OKT4^+/OKTK8^+$ de 0,1.

Ces deux rétrovirus ont été dénommés IDAV1 et IDAV2 respectivement, IDAV correspondant à l'abréviation de l'expression anglaise "Immune Deficiency Associated Virus" (Virus Associé à une Immuno Dépression). Des résultats d'une caractérisation partielle obtenus jusqu'à maintenant indiquent une similarité - sinon une identité - de IDAV1 et IDAV2 à LAV1, notamment du fait :

- des mêmes besoins ioniques et des mêmes spécificités de matrice de la reverse transcriptase,
- de la même morphologie dans des sections ultrafines,
- de la parenté antigénique des protéines p25 : le sérum d'un malade atteint de LAV1 a immunoprécipité la p25 obtenue à partir de IDAV1 et de IDAV2 ; réciproquement, un sérum provenant d'un malade infecté par IDAV2 a immunoprécipité la p25 de LAV1.

Un sérum de malade atteint de IDAV1 a semblé présenter un plus faible taux en anticorps et a donné une faible bande de précipitation pour les protéines p25 de IDAV1 et de LAV1. La protéine p25 de IDAV1 et de IDAV2 n'était pas reconnue par l'antisérum p24 de HTLV.

Ces similarités indiquent que tous ces trois isolats appartiennent au même groupe de virus.

La présente invention concerne de plus un procédé de diagnostic in vitro du SLA et du SIDA, qui comprend la mise en contact d'un sérum ou d'un autre milieu biologique provenant d'un malade faisant l'objet du diagnostic avec un extrait de virus tel que défini ci-dessus ou l'un quelconque des extraits purifiés préférés dénommés ci-dessus, et la détection de la réaction immunologique.

Des méthodes préférées mettent en jeu des titrages immuno-enzymatiques ou immunofluorescents, en particulier conformément à la technique ELISA. Les titrages peuvent être des dosages par immunofluorescence ou des dosages immuno-enzymatiques directs ou indirects.

Ainsi, la présente invention est également relative à des extraits de virus marqués quel que soit le type de marquage : enzymatique, fluorescent, radioactif etc.

De tels titrages comprennent par exemple :
- le dépôt de quantités déterminées de la protéine p25 purifiée conforme à la présente invention dans les puits d'une microplaque de titrage ;
- l'introduction dans ces puits de dilutions croissantes du sérum à diagnostiquer ;
- l'incubation de la microplaque ;

- le lavage soigneux de la microplaque ;
- l'introduction dans les puits de la microplaque d'anti-corps marqués spécifiques d'immunoglobulines du sang, le marquage étant réalisé par une enzyme choisie parmi celles qui sont capables d'hydrolyser un substrat de telle sorte que ce dernier subit alors une modification de son absorption des radiations, au moins dans une bande de longueurs d'ondes déterminée et
- la détection, de préférence de façon comparative par rapport à un témoin, de l'importance de l'hydrolyse du substrat en tant que mesure des risques potentiels ou de la présence effective de la maladie.

La présente invention est également relative à des nécessaires ou "kits" pour le diagnostic ci-dessus, lesquels comprennent :

- une protéine purifiée obtenue à partir des types de rétrovirus indiqués ci-dessus, cette protéine étant marquée, par exemple de façon radioactive, enzymatique ou immunofluorescente ;
- des anti-immunoglobulines humaines ou une protéine A (fixée de façon avantageuse sur un support insoluble dans l'eau tel que des billes d'agarose) ;
- un extrait de lymphocytes obtenus à partir d'une personne en bonne santé ;
- des tampons et, le cas échéant, des substrats pour la visualisation du marquage.

D'autres aspects de la présente invention apparaitront dans la description qui suit des procédures préférées d'isolement et de cultures du virus en question, des méthodes d'extraction préférées d un extrait approprié en tant que moyen de diagnostic, d'une technique de diagnostic préférée et des résultats qui peuvent ainsi être obtenus.

## 1. PROPAGATION DU VIRUS

Des lymphocytes T cultivés provenant soit du sang soit du cordon ombilical, soit également des cellules de moelle d'os de donneurs adultes en bonne santé, négatifs en ce qui concerne le virus, sont appropriés à la réalisation de la propagation du virus.

Il y a cependant une certaine variation d'un individu à l'autre en ce qui concerne la capacité des lymphocytes à faire croître le virus. Il est donc préférable de choisir un donneur en bonne santé adulte, qui n'a aucun anticorps vis-à-vis du virus et dont les lymphocytes ne libèrent spontanément ni le virus de façon répétée comme cela peut être détecté par l'activité de la reverse transcriptase (RT), ni des proteines d'expression virale.

Des lymphocytes du donneur sont obtenus et séparés par cytophorèse puis stockés à l'état congelé à -180°C dans de l'azote liquide, dans un milieu RPMI 1640, complété avec 50 % de sérum humain dépourvu de complément et 10 % de DMSO jusqu'au moment de l'utilisation.

Pour l'infection virale, on a mis les lymphocytes en culture (milieu RPMI 1640) avec de la phytohématoglutinine(PHA) à la concentration de $5.10^6$ cellules/ml pendant 3 jours.

Ensuite, on a enlevé le milieu et les cellules ont été remises dans une suspension virale (surnageant brut de lymphocytes produisant le virus, stocké à -80°C). Des conditions optimales correspondent à des concentrations de cellules/virus de $2.10^6$ cellules pour 5.000 à 10,000 cpm d'activité de RT, cette dernière étant déterminée de la façon décrite plus haut. Après 24 heures, on a centrifugé les cellules pour éliminer le virus non adsorbé et on les a remises en suspension dans un milieu de culture dépourvu de PHA et complété avec du TCGF dépourvu de PHA (Interleukin 2) : (0,5 - 1 U/ml, concentration finale), POLYBREN (Sigma) 2 $\mu$g/ml et de sérum de mouton anti - $\alpha$ interféron, inactivé à 56°C pendant 30 minutes (0,1 % d'un sérum qui est capable de neutraliser 7 U d'$\alpha$ interféron leucocytaire à une dilution de 1/100,000).

On a testé la production d'un virus tous les trois jours en déterminant l'activité de la RT sur des échantillons de 1 ml.

La présence de sérum anti-interféron est importante dans la production de virus : lorsque des lymphocytes sont infectés en l'absence d'antisérum d'$\alpha$ interféron humain, la production de virus, telle que titrée par l'activité de RT, est très faible ou retardée.Comme l'antisérum de mouton utilisé est suscité contre un $\alpha$ interféron leucocytaire partiellement purifié, préparé selon la technique Cantell, le rôle de composants autres que l'interféron ne peut pas être exclu.

La production de virus commence généralement entre le 9e et le 15e jour suivant l'infection et dure de 10 à 15 jours. En aucun cas, on a observé l'émergence d'une lignée permanente continue.

## 2. PURIFICATION DU VIRUS

Pour son utilisation dans le test ELISA, on a concentré le virus par précipitation avec 10 % de polyéthylèneglycol (PEG 6000) et on l'a centrifugé à deux reprises jusqu'à l'équilibre des bandes dans un gradient de sucrose de 2O à 6O %. La bande virale , de densité de 1,16, a été récupérée et utilisée en tant que telle pour des titrages ELISA.

Dans le cas du dosage par précipitation radio-immune RIPA on a trouvé qu'il est préférable de procéder à une purification dans des gradients isotoniques de métrizamide (vendu sous la marque commerciale NYCODENZ par Nyegaard, Oslo). La densité virale dans de tels gradients était très faible (1,1O - 1,11).

Le marquage métabolique avec la $^{35}$S-méthionine des cellules et du virus (RIPA) suivi par une électrophorèse sur gel de polyacrylamide ont été réalisés de la façon décrite ci-dessus, à part les modifications suivantes pour le RIPA : on a lysé le virus purifié dans du NYCODENZ dans 4 volumes de RIPA contenant 5OO U/ml d'aprotinine. On a effectué une incubation avec 5 µl de sérum à tester pendant 1 heure à 37°C puis 18 heures à +4°C. Une incubation supplémentaire des immunocomplexes avec des billes de protéine $^A$ SEPHAROSE a été réalisée pendant 3 heures à +4°C.

## 3. PREPARATION DE L'EXTRAIT DE VIRUS POUR DES TITRAGES ELISA

On a lysé le virus purifié dans un gradient de sucrose à la façon décrite ci-dessus dans du tampon RIPA (O,5 % de SDS) et on l'a déposé dans des puits de plaques de microtitrage (Nunc).

Des conditions préférées pour le titrage ELISA sont résumées ci-après.

Après addition à des puits en double de dilutions en série de chacun des sérums à tester, les IgGs fixées de façon spécifique sont révélées par de l'anti IgG humaine de chèvre associée à de la peroxydase. La réaction enzymatique est effectuée sur de l'ortho-phénylène-diamine comme substrat et lue avec un spectrophotomètre automatique à 492 nM.

Sur la même plaque, on a testé chaque sérum sur un antigène témoin (lysat cytoplasmique brut de lymphocytes T non infectés provenant du même donneur) afin d'éliminer une liaison non spécifique, qui peut être élevée avec certains sérums.

On a considéré que des sérums étaient positifs (anticorps contre le virus) lorsque la différence entre la D.O. obtenue avec l'antigène viral et la D.O obtenue avec l'antigène cellulaire témoin était d'au moins O,3O.

On décrit ci-après un test spécifique pour titrer la maladie mentionnée plus haut ou l'exposition à des risques de maladie.

### Méthode

Ce test ELISA sert à détecter et à titrer des anticorps sériques anti-rétrovirus du type LAV.

Il comprend la réalisation d'un test de compétition entre un antigène viral (cultivé sur des lymphocytes T) et un antigène témoin constitué par un lysat des mêmes lymphocytes cependant non infectés.

La liaison des anticorps aux deux antigènes est révélée par l'utilisation d'une antiglobuline humaine marquée par une enzyme, laquelle est elle-même révélée par l'addition d'un substrat correspondant.

### Préparation de l'antigène viral

Les cultures cellulaires qui sont utilisées sont des lymphocytes T d'origine humaine qui proviennent de :

. sang du cordon ombilical,
. moelle d'os,
. sang d'un donneur en bonne santé.

On utilise le surnageant d'une culture cellulaire infectée après avoir réalisé l'infection des cellules par le virus. Il est concentré par précipitation avec 1O % de PEG, puis purifié (deux ou trois fois) sur un gradient de sucrose (2O à 6O %) par ultracentrifugation à l'équilibre.

Les fractions virales sont rassemblées et concentrées par centrifugation à raison de 5O OOO tours par minute pendant 6O minutes.

Le virus sédimenté est repris dans un volume minimum d'un tampon NTE à pH 7,4 (Tris O,O1 M, NaCl O,1 M, EDTA O,OO1 M).

La concentration en protéine est déterminée par la méthode de Lowry.

Le virus est ensuite lysé par un tampon (RIPA + SDS), (O,5 % du produit final) pendant 15 minutes à 37°C.

### Préparation de l'antigène témoin

Les lymphocytes non infectés sont cultivés dans les conditions indiquées ci-dessus pendant de 5 à 1O jours. Ils sont centrifugés à basse vitesse et lysés dans le tampon RIPA en présence de 5 % du produit commercialisé sous la marque de ZYMOFREN (Spécia) (500 $\mu$/ml). Après un repos de 15 minutes à 4°C avec agitations fréquentes dans un dispositif VORTEX, le lysat est centrifugé à 10 000 tours par minute. Le surnageant constitue l'antigène témoin. Sa concentration en protéine est mesurée par la méthode de Lowry.

Réactifs

    1 - Plaque = NUNC - spécialement contrôlées pour ELISA
    2 - Tampon PBS : pH 7,5
    3 - TWEEN 20
    4 - Tampon de carbonate :
    pH = 9,6
    ($CO_3Na_2$ = 0,2 M
    ($CO_3HN_a$ = 0,2 M
    5 - Sérum de veau non fétal : qui est stocké à l'état congelé (BIOPRO),
    6 - Albumine de sérum bovin (BSA) SIGMA (fraction V)
    7 - Anti IgG (H + L) humaine marquée avec de la péroxydase PASTEUR, dans des tubes de 1 ml conservés à 4°C
    8 - Tampon de lavage = Tampon PBS, pH 7,5 + 0,05 % TWEEN 20
    La dilution du conjugué est effectuée à la dilution indiquée dans le tampon PBS + TWEEN 20 (0,05 %) + albumine bovine 0,5 g à 100 ml
    9 - Tampon de dilution des sérums : Tampon PBS + 0,05 % TWEEN 20 + 0,5 g BSA Albumine de sérum bovin par 100 ml
    10 - Substrat = OPD
      . Tampon de citrate pH = 5,6 citrate trisodique
      ($C_6H_5Na_4O_3$, $2H_2O$), 0,05 M ;
      acide citrique
      ($C_6H_8O_7$, 1 $H_2O$), 0,05 M.
      . peroxyde d'hydrogène = à 30 % (110 volumes) - utilisé à 0,03 % lorsqu'on emploie le tampon de citrate.
      . Orthophénylène diamine = SIGMA
      75 mg par 25 ml de tampon - qui est dilué dans le tampon sans préparation.

Préparation des plaques

On a utilisé des plaques comprenant 96 puits en forme de U (NUNC = ELISA). Elles comprennent 12 rangées de 8 puits chacune, numérotées de 1 à 12.
La distribution des antigènes est la suivante :
- on dépose 100 $\mu$l de l'antigène viral, dilué dans le tampon de carbonate à pH 9,6, dans chacun des puits des rangées (marquées ⊕ )
    1 - 2 - 5 - 6 - 9 - 10
- on dépose 100 $\mu$l de l'antigène témoin, dilué dans le tampon de carbonate à pH 9,6 dans chacun des puits des rangées (marquées ⊖ )
    3 - 4 - 7 - 8 - 11 - 12.
La dilution de l'antigène viral est titrée à chacune des productions virales. On teste plusieurs dilutions d'antigène viral et on les compare à des témoins connus positifs et négatifs (à plusieurs dilutions) et à de l'anti-IgG humaine marquée avec de la péroxydase, ce dernier étant également testé à plusieurs dilutions.
En règle générale, la concentration protéique de la préparation est de 5 à 2,5 $\mu$g/ml.
On utilise la même concentration protéique pour l'antigène témoin.
Les plaques sont fermées avec un couvercle plastique et incubées pendant 16 heures à 4°C.
Elles sont ensuite mis une fois dans de l'eau distillée et centrifugées. Les puits sont alors remplis avec 300 $\mu$l de sérum de veau non fétal à 20 % dans du tampon PBS.
L'incubation dure 2 heures à 37°C (plaques recouvertes).
Les plaques sont lavées à 3 reprises dans du tampon PBS avec du TWEEN 20, 0,05 % tampon (PBS-tw) :
    . Premier lavage 300 $\mu$l
    . deuxième et troisième lavage 200 $\mu$l par puits.

Les plaques sont soigneusement séchées et refermées avec un film plastique adhésif. Elles peuvent être stockées à -80° C.

Réaction ELISA : Dosage du titre en anticorps

Après décongélation, les plaques sont lavées à 3 reprises dans du PBS-TWEEN. Elles sont soigneusement séchées.

Les sérums témoins positifs et négatifs ainsi que les sérums testés sont dilués d'abord dans des tubes,avec du PBS-TWEEN contenant 0,5 % d'albumine de boeuf.

La dilution choisie est de 1/40.
- on dépose 100 μl de chaque sérum en double sur l'antigène viral et en double sur l'antigène témoin.
- on procède de même avec les sérums dilués positifs et négatifs.
- on introduit 100 μl de PBS + TWEEN + albumine de sérum bovin dans deux puits ⊕ et dans deux puits ⊖ pour former les témoins conjugués.

Les plaques munies de leurs couvercles sont incubées pendant 1 heure 30 à 37° C.

Elles sont lavées à 4 reprises dans du PBS + TWEEN à 0,05 %.
- on dépose 100 μl d'anti-IgG (marqué avec de la peroxydase) à la dilution choisie dans chaque puits et l'on incube à 37° C.

Les plaques sont à nouveau lavées à 5 reprises avec du tampon (PBS + TWEEN). Elles sont soigneusement séchées.

La révélation de la réaction enzymatique est effectuée au moyen d'un substrat d'orthophénylène-diamine (0,05 % dans un tampon de citrate, pH 5,6, contenant 0,03 % de $H_2O_2$).

100 μl de substrat sont distribués dans chaque puits.

Les plaques sont laissées dans une pièce obscure pendant 20 minutes à la température du laboratoire.

La lecture est effectuée sur un spectrophotomètre (pour microplaques) à 492 nm.

On a estimé que des sérums contenant des anticorps vis-à-vis du virus sont ceux qui fournissent une DDO (Différence Densité Optique = densité optique de l'antigène viral diminuée de la densité optique de l'antigène témoin) égale ou supérieure à 0,30.

Cette technique permet un titrage qualitatif aussi bien que quantitatif. A ce propos, il est possible soit d'utiliser plusieurs dilutions du sérum à tester, soit de comparer une dilution du sérum avec une gamme de témoins testés dans les mêmes conditions.

Le tableau ci-après fournit les premiers résultats des études sérologiques effectuées pour les anticorps de LAV, réalisées à l'aide de la méthode de titrage ELISA décrite plus haut.

Premiers résultats des études sérologiques pour

des anticorps de LAV en FRANCE

| | Total examiné | test ELISA-LAV | | Test ELISA-HTLV1** (Biotech.) | |
|---|---|---|---|---|---|
| | | positif | % de posi-tifs | positif | % de posi-tifs |
| malades atteints de lymphadéno-pathie* | 35 | 22 | (63) | 5*** | (14) |
| homosexuels en bonne santé | 40 | 7 | (17) | 1 | (3) |
| Population témoin | 54 | 1 | (1,9) | O | (<2,6) |

* 28 homosexuels

   3 Haïtiens (1 femme)

   4 toxicomanes (2 femmes)

** Le nombre de sérums positifs est probablement surestimé dans cet essai, puisqu'on n'a pû faire aucun contrôle de liaisons non spécifiques.

*** Parmi les 5 personnes ayant fourni un résultat positif dans le test ELISA-HTLV[1], 3 étaient nées à Haïti, 1 avait séjourné longtemps à Haïti et 1 avait effectué plusieurs voyages aux Etats-Unis.

Toutes ces personnes avaient également des anticorps contre le LAV.

Le tableau ci-dessus montre nettement la forte prédominance des anticorps de LAV chez les malades homosexuels atteints de SLA, la très faible incidence dans la population normale et également un développement modéré de l'infection par le virus chez des homosexuels encore en bonne santé. Dans le dernier groupe, tous les individus ayant une réponse positive avaient un grand nombre de partenaires ( > 50 par année). L'apparition des anticorps de HTLV était très faible dans l'ensemble des groupes (déterminée à l'aide du test ELISA commercial (Biotech.)). Les groupes des malades atteints du SIDA ont donné des résultats moins faciles à interpréter : environ 20 % avaient des anticorps de LAV, mais certains des sérums ont été prélevés lors d'une étape très tardive de la maladie, avec une possible négativation de la réponse humorale.

Il faut de plus indiquer que les lymphocytes de tous les malades atteints du SLA ne produisent pas des quantités détectables de virus du type LAV. En particulier, des cellules de ganglions lymphatiques provenant de 6 autres malades atteints du SLA ont été mises en culture et testées pour la production de virus de la façon décrite pour le malade 1. On n'a pû détecter aucune libération de virus par l'activité de RT. Cependant, on a pû détecter une protéine p25 reconnue par le sérum du premier malade dans des extraits cytoplasmiques des cellules T marquées avec de la $^{35}$S-méthionine dans 3 autres cas. Cela suggère une expression partielle d'un virus similaire dans de tels cas. De plus, tous ces malades (6/6) avaient des anticorps contre des protéines p25 de LAV, indiquant qu'ils avaient tous été infectés avec un virus analogue ou identique.

Il est intéressant de constater, dans des lymphocytes de l'un des malades (malade n° 2) qu'il y avait une immunoprécipitation faible mais nette d'une bande de dimension analogue (p24-p25) avec de l'antisérum de chèvre spécifique du HTLV1. De façon analogue, le sérum du malade avait des anticorps contre à la fois HTLV et LAV, suggérant une double infection par les virus. De tels cas ne semblent pas fréquents.

La présente invention se rapporte enfin à des réactifs biologiques qui peuvent être formés par les extraits de LAV contenant la protéine p25 ou par la protéine p25 purifiée,en particulier pour la production d'anticorps dirigés contre la p25 chez les animaux ou d'anticorps monoclonaux. Ces anticorps sont susceptibles de former d'utiles outils dans l'étude ultérieure de déterminants antigéniques des virus LAV où des virus apparentés à LAV.

Il est bien évident que les dénominations OKT qui ont été utilisées à l'occasion de la désignation de certains sous-ensembles de lymphocytes ou d'anticorps monoclonaux apparentés pour plus de facilité, ne doivent en aucun cas être opposés à la validité d'une quelconque marque commerciale correspondante, qu'elle soit déposée ou non par son propriétaire.

Il doit de plus être mentionné que l'extraits viral enrichi en protéine p25 peut également être définis par référence à leur relation ou similitude immunologique avec la protéine p25, telle qu'on peut l'obtenir à partir des souches LAV1, IDAV 1 ou IDAV 2. Ainsi, toute fraction protéique qui est capable de donner des profils de réaction immunologique analogues à ceux que donne la protéine purifiée de LAV1, IDAV1 ou IDAV2 avec les mêmes sérums, doit être considérée comme équivalente à ceux-ci et, en conséquence, être englobée dans la portée des revendications qui suivent. Une conclusion similaire s'étend évidemment aux moyens de diagnostic (procédé et "kits") qui mettent en jeu des fractions protéiques équivalentes.

Le virus LAV1 a été déposé à la "Collection Nationale des Cultures de Micro-organismes" (C.N.C.M.) sous le numéro I-232 le 15 juillet 1983 et les virus IDAV1 et IDAV2 ont été déposés à la C.N.C.M. le 15 septembre 1983 sous les numéros I-240 et I-241, respectivement. L'invention englobe également les proteines p25 ou fractions protéiques des mutants ou des variantes des souches déposées ci-dessus dans la mesure où ils possèdent pratiquement les mêmes propriétés immunologiques.

**Revendications**

1. Méthode pour le diagnostic in vitro d'un SLA ou du SIDA, caractérisée
   - en ce que l'on met en contact un sérum ou un autre milieu biologique provenant du malade faisant l'objet du diagnostic, avec une protéine p25 purifiée, présentant un poids moléculaire d'environ 25.000, immunologiquement distincte des protéines p19 et p24 de HTLV1, obtenue à partir d'un extrait de l'un des rétrovirus déposés à la C.N.C.M. sous les numéros I-232, I-240 et I-241 ou de l'un de leurs mutants ou variants, ou avec une fraction protéique de cette protéine p25 purifiée et susceptible de donner lieu à une réaction immunologique avec des anticorps dirigés contre la protéine p25 purifiée
   - en ce que l'on détecte la réaction immunologique éventuelle entre cette protéine et ce sérum ou autre milieu biologique.

2. Kit pour la détection in vitro d'un SLA ou du SIDA, dans des sérums provenant de malades, caractérisé en ce qu'il comprend :
   - une protéine p25 purifiée présentant un poids moléculaire d'environ 25.000, immunologiquement distincte des protéines p19 et p24 de HTLV1, obtenue à partir d'un extrait de l'un des rétrovirus déposés à la C.N.C.M. sous les numéros I-232, I-240 et I-241 ou de l'un de leurs mutants ou variants, ou une fraction protéique de cette protéine p25 purifiée et susceptible de donner lieu à une réaction immunologique avec des anticorps dirigés contre la susdite protéine p25 purifiée, ladite protéine ou fraction protéique étant marquée,
   - des anti-immunoglobulines humaines,
   - un extrait de lymphocytes obtenu à partir d'une personne en bonne santé,
   - des tampons et, le cas échéant, des substrats pour la visualisation du marquage,
   - des moyens pour détecter le conjugué marqué résultant de la réaction immunologique entre la protéine ou fraction protéique marquée et les sérums.

3. Kit pour le dosage de SIDA ou d'un SLA, caractérisé en ce qu'il comprend :
   - une protéine p25 purifiée présentant un poids moléculaire d'environ 25.000, immunologiquement distincte des protéines p19 et p24 de HTLV1, obtenue à partir d'un extrait de l'un des rétrovirus déposés à la C.N.C.M. sous les numéros I-232, I-240 et I-241 ou de l'un de leurs mutants ou variants, ou une fraction protéique de cette protéine p25 purifiée et susceptible de donner lieu à une réaction immunologique avec des anticorps dirigés contre la susdite protéine p25 purifiée, ladite protéine ou fraction protéique étant marquée,
   - des anti-immunoglobulines humaines,
   - un extrait de lymphocytes obtenu à partir d'une personne en bonne santé,
   - des tampons et, le cas échéant, des substrats pour la visualisation du marquage,
   - des moyens pour doser le conjugué marqué résultant de la réaction immunologique entre la protéine ou fraction protéique marquée et le milieu biologique soumis au dosage.

## Claims

1. A method for the in vitro diagnosis of a LAS or of AIDS, characterized,
   - in that one brings into contact a serum or another biological medium obtained from a patient who is subject to said diagnosis, with a purified p25 protein, which exhibits a molecular weight of about 25,000, immunologically distinct from the p19 and p24 proteins of HTLV1, obtained from an extract of one of the retroviruses deposited at the C.N.C.M. under numbers I-232, I-240 or I-241 or of one of their mutants or variants, or with a proteic fraction of said purified p25 protein and which is liable of providing an immunological reaction with antibodies directed against the purified p25 protein
   - in that one detects the possible immunological reaction between said protein and said serum or other biological medium.

2. A kit for the in vitro detection of a LAS or of AIDS in serums originating from patients, characterized in that it comprises:
   - a purified p25 protein exhibiting a molecular weight of about 25,000, immunologically distinct from the p19 and p24 proteins of HTLV1, obtained from the extract of one of the retroviruses deposited at the C.N.C.M. under numbers I-232, I-240 and I-241 or of one of their mutants or variants, or a proteic fraction of said purified p25 protein and capable of giving rise to an immunological reaction with antibodies directed against said purified p25 protein, said protein or proteic fraction being labelled,
   - human anti-immunoglobulins,
   - an extract of lymphocytes obtained from a healthy person,
   - buffers and, possibly, substrates for the visualization of the labelling,
   - means for detecting the labelled conjugate resulting from the immunological reaction between the labelled protein or proteic fraction and the serums.

3. A kit for the dosage of AIDS or of a LAS, characterized in that it comprises:
   - a purified p25 protein exhibiting a molecular weight of about 25,000, immunologically distinct from the p19 and p24 proteins of HTLV1, obtained from an extract of one of the retroviruses deposited at the C.N.C.M. under numbers I-232, I-240 and I-241 or from one of their mutants or variants, or with a proteic fraction of said purified p25 protein and liable of giving rise to an immunological reaction with antibodies directed against said purified p25 protein, said protein or proteic fraction being labelled,
   - human anti-immunoglobulins,
   - a lymphocytes extract obtained from said healthy person,
   - buffers and, possibly, substrates for the visualization of the labelling
   - means for dosing the labelled conjugate resulting from the immunological reaction between the labelled protein or proteic fraction and the biological medium subjected to the dosage.

## Patentansprüche

1. Verfahren zur in-vitro-Diagnose eines Lymphadenopathie-Syndroms (SLA) oder eines erworbenen Immundepressions-Syndroms (AIDS), dadurch gekennzeichnet, daß
   - man ein Serum oder eine andere biologische Probe eines Kranken mit einem gereinigten p25-Protein, das ein Molekulargewicht von etwa 25.000 aufweist, sich immunologisch von den p19- und p24-Proteinen des HTLV1 unterscheidet, aus einem Extrakt aus einem der Retroviren, die bei der C.N.C.M. ("Collection Nationale des Cultures de Micro-organismes") unter den Nummern I-232, I-240 und I-241 hinterlegt sind, oder aus einer ihrer Mutanten oder Varianten erhalten ist, oder mit einem Proteinbruchstück dieses gereinigten p25-Proteins, das fähig ist, eine immunologische Reaktion mit gegen das gereinigte p25-Protein gerichteten Antikörpern zu veranlassen, in Kontakt bringt, und

man die eventuelle immunologische Reaktion zwischen diesem Protein und diesem Serum oder anderen biologischen Proben nachweist.

2. Kit zum in-vitro-Nachweis eines Lymphadenopathie-Syndroms (SLA) oder eines erworbenen Immundepressions-Syndroms (AIDS) in Seren von Kranken, dadurch gekennzeichnet, daß er enthält:
- ein gereinigtes p25-Protein, das ein Molekulargewicht von etwa 25.000 aufweist, sich immunologisch von den p19- und p24-Proteinen des HTLV1 unterscheidet, aus einem Extrakt aus einem der Retroviren, die bei der C.N.C.M. unter den Nummern I-232, I-240 und I-241 hinterlegt sind, oder aus einer ihrer Mutanten oder Varianten erhalten ist, oder ein Proteinbruchstück dieses gereinigten p25-Proteins, das fähig ist, eine immunologische Reaktion mit gegen das gereinigte p25-Protein gerichteten Antikörpern zu veranlassen, wobei dieses Protein oder das Proteinbruchstück markiert ist,
- menschliche Anti-Immunglobuline,
- einen Extrakt von Lymphozyten, die von einer gesunden Person stammen,
- Puffer und gegebenenfalls Substrate zum Sichtbarmachen der Markierung,
- Mittel zum Nachweis des markierten Konjugats, das sich aus der immunologischen Reaktion zwischen dem Protein oder dem markierten Proteinbruchstück und den Seren ergibt.

3. Kit zur Dosierung eines erworbenen Immundepressions-Syndroms (AIDS) oder eines Lymphadenopathie-Syndroms (SLA), dadurch gekennzeichnet, daß er enthält:
- ein gereinigtes p25-Protein, das ein Molekulargewicht von etwa 25.000 aufweist, sich immunologisch von den p19- und p24-Proteinen des HTLV1 unterscheidet, aus einem Extrakt aus einem der Retroviren, die bei der C.N.C.M. unter den Nummern I-232, I-240 und I-241 hinterlegt sind, oder aus einer ihrer Mutanten oder Varianten erhalten ist, oder ein Proteinbruchstück dieses reinen p25-Proteins, das fähig ist, eine immunologische Reaktion mit gegen das oben genannte gereinigte p25-Protein gerichteten Antikörpern zu veranlassen, wobei dieses Protein oder die Proteingruppe markiert ist,
- menschliche Anti-Immunglobuline,
- einen Extrakt von Lymphozyten, die von einer gesunden Person stammen,
- Puffer und gegebenenfalls Substrate zum Sichtbarmachen der Markierung,
- Mittel zum Dosieren des zu dosierenden markierten Konjugats, das aus der immunologischen Reaktion zwischen dem Protein oder dem markierten Proteinbruchstück und der biologischen Probe entsteht.

mesure de l'activité de la reverse transcriptase sur des fractions successives du gradient de sucrose,

mesure de la substance marquée avec la [³H] uridine, qui peut être précipitée par un acide,

variation de la densité du gradient.